# EUROPEAN PATENT SPECIFICATION

(11) **EP 3 563 901 B1**
(45) Date of publication and mention of the grant of the patent: **10.09.2025**
(21) Application number: 19161639.0
(22) Date of filing: 08.03.2019
(51) Int. Cl.: A61M 39/06, A61B 17/34, A61B 1/00, A61B 1/018

(54) **A MEDICAL VALVE WITH A VARIABLE DIAMETER SEAL**
EIN MEDIZINISCHES VENTIL MIT EINER DICHTUNG MIT VARIABLEM DURCHMESSER
VALVE MÉDICALE DOTÉE D'UN JOINT À DIAMÈTRE VARIABLE

(30) Priority: 30.04.2018 US 201815966173
(43) Date of publication of application: 06.11.2019
(73) Proprietor: Freudenberg Medical, LLC, Carpinteria, CA 93013 (US)
(72) Inventor: Furnish, Greg, Louisville, Kentucky 40206 (US); Appling, Anthony, Crestwood, Kentucky 40014 (US); Morris, Ben, Jeffersonville, Indiana 47130 (US); Zeis, Timothy, Charlestown, Indiana 47111 (US)
(74) Representative: Manitz Finsterwald Patent- und Rechtsanwaltspartnerschaft mbB

(56) References cited:
- EP-A1- 2 965 685
- EP-A1- 3 088 039
- US-A- 5 882 345
- US-A1- 2006 241 671
- US-B1- 6 572 590

## Description

### CROSS-REFERENCE TO RELATED APPLICATIONS

This application is a continuation-in-part of United States Patent Application No. 15/483,089 filed on April 10, 2017, which is a continuation of United States Patent Application No. 14/326,593 filed on July 9, 2014, now U.S. Patent No. 9,616,213

### BACKGROUND OF THE INVENTION

### 1. Field of the Invention

The present disclosure relates generally to medical devices and procedures. In particular, the present disclosure relates to hemostatic valves and systems, and methods of using the same.

### 2. Description of the Prior Art

This section provides background information related to the present disclosure which is not necessarily prior art.

Numerous procedures have been developed that involve the percutaneous insertion of a medical device into a body vessel of a patient, with the medical device being introduced into the vessel by a variety of known techniques. Each of these procedures must control the flow of bodily fluids when the medical device is inserted into the body vessel. Accordingly, medical valves, such as hemostatic valves, iris valves, laproscopic ports, or the like, are often used to limit or prevent blood loss during the procedure.

Hemostatic valves often incorporate a disk valve to control fluid flow through the medical device. However, disk valves are subject to deformation with both time and use, and often can tear or become dislodged during insertion and/or withdrawal of the medical device. Furthermore, disk valves are not designed to provide an effective seal across a wide range of differently sized medical devices. Although the disk valve can be modified to accommodate these situations, such as with increased tensile and/or elongation properties, this modification leads to increased resistance, and thus require the use of excessive force, when the medical device is inserted and withdrawn through the disk valve.

Iris valves can include an elastomeric sleeve that is disposed within a valve body and which is interconnected to a rotatable cap. When the cap is rotated in a first direction, an opening extending through the elastomeric sleeve is opened. Conversely, when the cap is rotated in a second opposite direction, the elastomeric sleeve is twisted and constricted to effectuate a closure of the elastomeric sleeve. However, if the operator stops the rotation, the elastomeric sleeve can revert, or recoil, back to the open position. Additionally, even when the elastomeric sleeve is held in the closed position, gaps or channels extend therethrough as a result of the twisting or infolding required to effectuate a closure. Accordingly, fluid can leak through the iris valve in the closed position. Further, the continuous twisting and constricting of the elastomeric sleeve leads to wear of the sleeve, such as through tearing.

The drawbacks associated with the existing medical valves are further exemplified when one considers that a single medical valve often is used to insert multiple medical devices during a single procedure. For example, a hemostatic valve may be used first for introducing a delivery catheter, followed by an interventional catheter. In this example, the hemostatic valve must be able to provide a hemostatic seal under a variety of conditions, i.e., accommodate a variety of different sized medical devices. Additionally, the hemostatic valve device must be able to quickly adjust to use of each of these different medical devices, otherwise significant fluid loss can occur through the medical valve.

Documents EP 3 088 039 A1 and US 6,572,590 B1 (D2) disclose medical valve assemblies according to the preamble of claim. Documents US 2006/0241671 A1 and US 5,882,345 show further examples of valve assemblies

### SUMMARY OF THE INVENTION

This section provides a general summary of the disclosure and is not intended to be a comprehensive disclosure of its full scope, aspects, objectives, and/or all of its features. The invention is defined by the appended claims.

A medical valve assembly for use in inserting a medical device into a body vessel of a patient includes a tube extending between a first tube end and a second tube end along an axis. A plunger plate extends radially from the second tube end and a valve housing surrounds the tube about the second tube end. The valve housing extends from a first valve housing end to a second valve housing end and includes a flange extending radially inwardly from the second valve housing end, with the flange disposed in spaced relationship with respect to the plunger plate so as to define a distance dimension therebetween. An elastomeric seal is compressed between the plunger plate and the flange and has an inner surface defining inner diameter for use in establishing a variable seal of the medical valve assembly. A coil spring is disposed within said valve housing and is compressed between said first valve housing end and said plunger plate for applying a compression force to said plunger plate for compressing said elastomeric seal from a non-compressed condition to a compressed condition to decrease said inner diameter and establish a closed condition of the medical valve assembly.. Said inner surface of said elastomeric seal has in said non-compressed condition a plurality of planar portions and a plurality of radiused portions as viewed in cross-section taken along a plane which extends perpendicularly to said axis and through said elastomeric seal with each of the plurality of radius portions being convex relative to said axis and adjacent ones of said plurality of planar portions interconnected with one of said plurality of radiused portions.

Further areas of applicability will become apparent from the description provided herein. The description and specific examples in this summary are intended for purposes of illustration only and are not intended to limit the scope of the present disclosure.

### BRIEF DESCRIPTION OF THE DRAWINGS

The drawings described herein are for illustrative purposes only of selected embodiments, and are not all possible implementations and thus are not intended to limit the scope of the present disclosure.
Figure 1 is an environmental view of a first embodiment of a medical valve constructed in accordance with the principles of the present disclosure and illustrating a user interacting therewith;
Figure 2 is an environmental view of a second embodiment of the medical valve constructed in accordance with the principles of the present disclosure and illustrating the user interacting therewith;
Figure 3 is a perspective view of the first embodiment of the medical valve illustrating a scissor-type manual actuator;
Figure 4A is a cross-sectional view of the first embodiment of the medical valve illustrating a closed condition;
Figure 4B is a cross-sectional view of the first embodiment of the medical valve illustrating an open condition;
Figure 5 is a partial view taken from Figure 3 illustrating an elastomeric seal of the medical valve;
Figure 6 is a cross-sectional view of the first embodiment of the medical valve illustrating an alternative arrangement for the manual actuator;
Figure 7 is a perspective view of the first embodiment illustrating a detachable cap disposed over a pair of lever arms associated with the scissor-type manual actuator;
Figure 8 is a perspective view of the second embodiment of a medical valve constructed in accordance with the present disclosure;
Figure 9 is a cross-sectional view of the second embodiment shown in Figure 8;
Figure 10 is a perspective view of the first embodiment illustrating an alternative arrangement of the scissor-type manual actuator;
Figure 11A is a cross-sectional view of the first embodiment of the medical valve illustrating an alternative arrangement of the elastomeric seal in the closed, or compressed, condition;
Figure 11B is a cross-sectional view of the first embodiment of the medical valve illustrating the alternative arrangement of the elastomeric seal in the open, or non-compressed, condition;
Figure 12 is a cross-sectional view of the alternative arrangement of the elastomeric seal in the open, non-compressed condition;
Figure 13 is a perspective, cross-sectional view of the elastomeric seal in the open, non-compressed condition, taken along line 13-13 of Figure 12 to illustrate an inner surface of the elastomeric seal having a plurality of planar portions and a plurality of radiused portions; and
Figure 14 is a perspective, cross-sectional view of the alternative arrangement of the elastomeric seal of Figure 13 but shown in the closed, compressed condition to illustrate the inner surface compressed or closed along the plurality of planar portions.

### DESCRIPTION OF THE ENABLING EMBODIMENTS

Example embodiments will now be described more fully with reference to the accompanying drawings. The example embodiments are provided so that this disclosure will be thorough and fully convey the scope to those skilled in the art. Numerous specific details are set forth such as examples of specific components, devices, mechanisms, assemblies, and methods to provide a thorough understanding of various embodiments of the present disclosure. It will be apparent to those skilled in the art that specific details need not be employed, that example embodiments may be embodied in many different forms, and that neither should be construed to limit the scope of the disclosure. With this in mind, the present disclosure is generally directed to medical valve assemblies of the type used to introduce and withdrawal a medical device (i.e., a guide wire, catheter, stent, filter, etc.) into a body vessel of a patient. In particular, each of the medical valve assemblies of the present disclosure incorporate a variable seal arrangement and a manually-operable actuator for controlling an entry dimension of the variable seal arrangement.

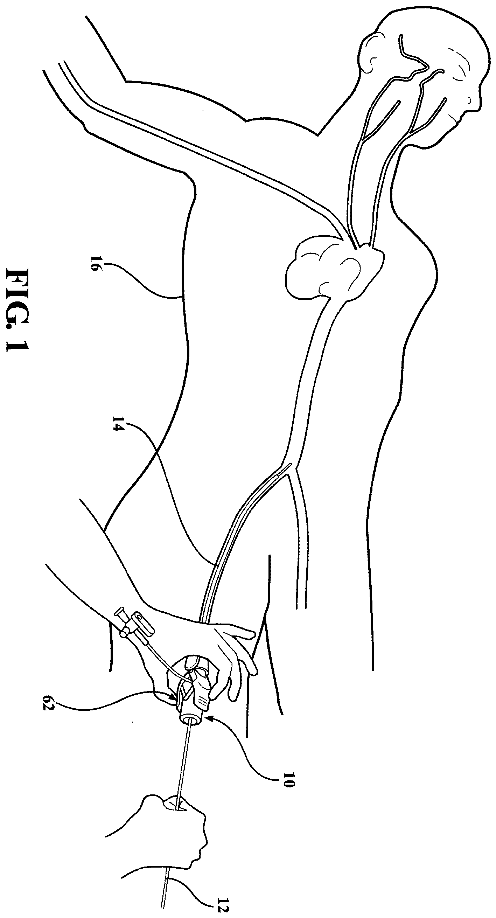

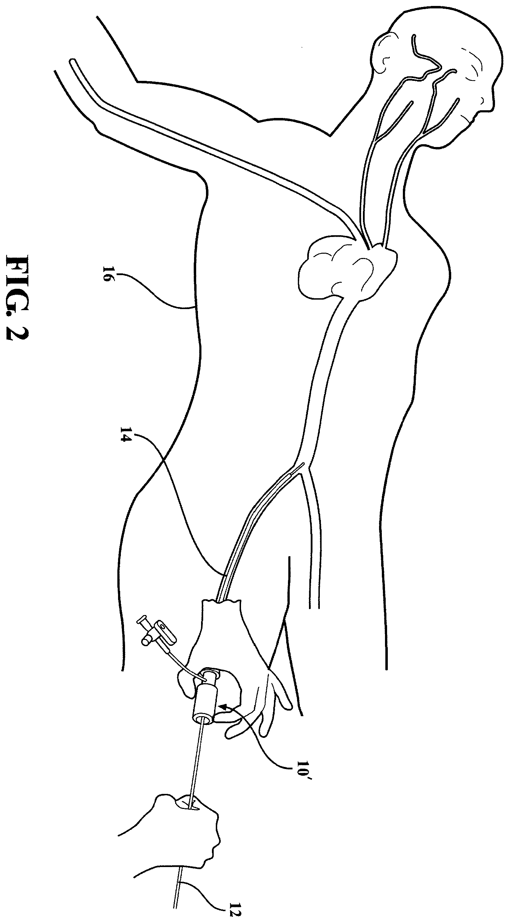

Referring to the Figures, wherein like numerals indicate corresponding parts throughout the several views, an environmental view of a first embodiment of a medical valve assembly **10** and a second embodiment of a medical valve assembly **10'** is generally shown in Figures 1 and 2, respectively. As illustrated therein, each medical valve assembly **10, 10'** is of the type for use with a medical device **12,** such as a guide wire, catheter, stent, filter, vessel occlusion device, or the like. As will be explained in more detail below, as the medical device **12** is inserted and guided through the medical valve assembly and into a body vessel **14** of a patient **16,** a user can manually actuate or interact with the medical valve assembly to effectuate a variable seal with a variety of different sized medical devices **12.**

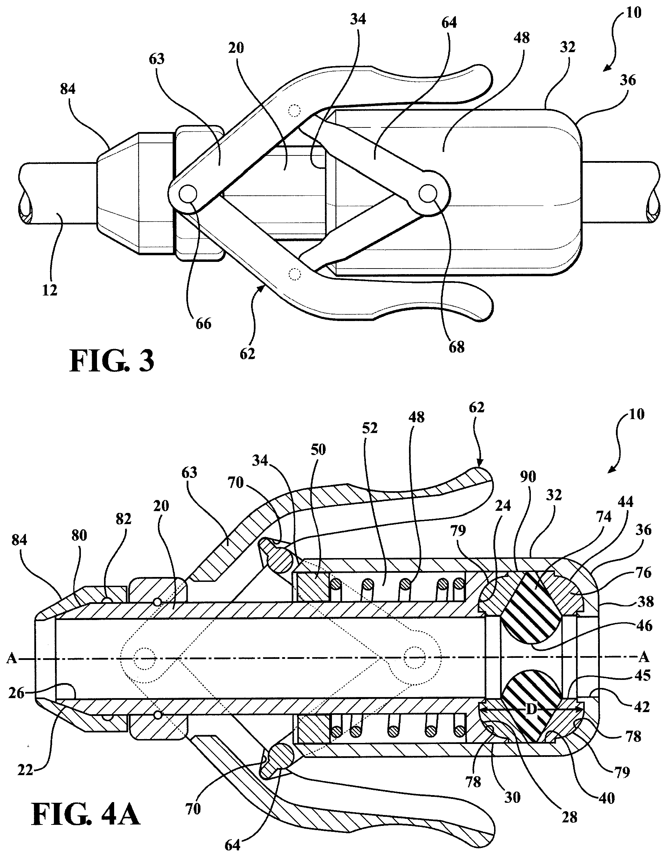

As best shown in Figures 4A, 4B, 9, 11A and 11B, the medical valve assemblies **10, 10'** each include a tube **20** extending between a first tube end **22** and a second tube end **24** to define a passageway **26** extending longitudinally along an axis **A** between the ends **22, 24,** with the passageway **26** being sized to receive a variety of differently sized medical devices **12.** In this instance, the first tube end **22** is a distal tube end and the second tube end **24** is a proximal tube end **24.** A plunger plate **28** extends radially from the second tube end **24** to define an outer plunger plate surface **30** extending in spaced and parallel relationship to the axis **A.** A valve housing **32** is disposed in surrounding relationship with the tube **20** about the second tube end **24** and extends from a first valve housing end **34** to a second valve housing end **36** to overlay the outer plunger plate surface **30**. In this instance, the first valve housing end **34** is a distal valve housing end and the second valve housing end **36** is a proximal valve housing end **36.** As best shown in Figures 4A, 4B, 9, 11A and 11B, the valve housing **32** is disposed in spaced and parallel relationship with the tube **20** between the first valve housing end **34** and the plunger plate **28.**

The valve housing **32** includes a flange **38** extending radially inwardly from the second valve housing end **36.** The flange **38** is disposed in spaced relationship with the plunger plate **28** to define a distance dimension **D,** as well as a cavity **40**, extending therebetween. The flange **38** also defines an opening **42** aligned on the axis **A** and that is sized to receive a variety of differently sized medical devices **12.** An elastomeric seal **44** is installed in the cavity **40** and normally is pre-loaded or compressed between the plunger plate **28** and the flange **38.** The elastomeric seal **44** has an inner surface **45** which is used to establish a variable seal of the medical valve assembly **10, 10'.** In both of the first and second embodiments of the medical valve assembly **10, 10',** one of the valve housing **32** or the tube **20** is axially movable relative to the other to vary the distance dimension **D** between the plunger plate **28** and the flange **38** for effectuating an adjustment of an inner diameter **46** as defined by the inner surface **45** of the elastomeric seal **44.** In other words, the axial movement of one of the valve housing **32** or the tube **20** relative to the other results in a change in the compression load exerted on the elastomeric seal **44** which, in turn, allows the inner diameter **46** defined by the inner surface **45** of the elastomeric seal **44** to be varied or adjusted in size. As best shown in Figures 4A, 4B, 9, 11A and 11B, when the valve housing **32** or the tube **20** is axially moved, the plunger plate **28** or the valve housing **32** axially slides relative to the other along the outer plunger plate surface **30**. In other words, the outer plunger plate surface **30** guides a sliding axial movement between the valve housing **32** and the tube **20**.

As best shown in Figures 4A, 4B, 9, 11A, and 11B, a compression member **48, 54** is disposed within the valve housing **32** and is compressed against the plunger plate **28** for normally closing or decreasing the inner diameter **46** through a compression of the elastomeric seal **44** to establish a closed position or condition of the elastomeric seal **44.** As a result, the compression member **48, 54** is arranged to effectuate a closing or decreasing of the inner diameter **46** of the elastomeric seal **44** to establish a closed condition of the medical valve assembly **10, 10'.** In its closed condition, the elastomeric seal **44** completely isolates or seals the opening **42** of the valve housing **32** from the passageway **26** of the tube **20**. The valve housing **32** or the tube **20** is then axially movable relative to the other to alter a distance **D** between the flange **38** and the plunger plate **28** and shift the medical valve assembly **10, 10'** from the closed condition to an open/operative condition. The altered or varied distance **D** between the flange **38** and the plunger plate **28** allows the elastomeric seal **44** to expand, and as a result, the inner diameter **46** of the elastomeric seal **44** is expanded or increased to move the elastomeric seal **44** from its closed position to an open position. With the elastomeric seal **44** in its open position, the medical device **12** is positioned to be inserted serially through the opening **42,** the inner diameter **46** of the elastomeric seal **44** and the passageway **26** of the medical valve assembly **10.**

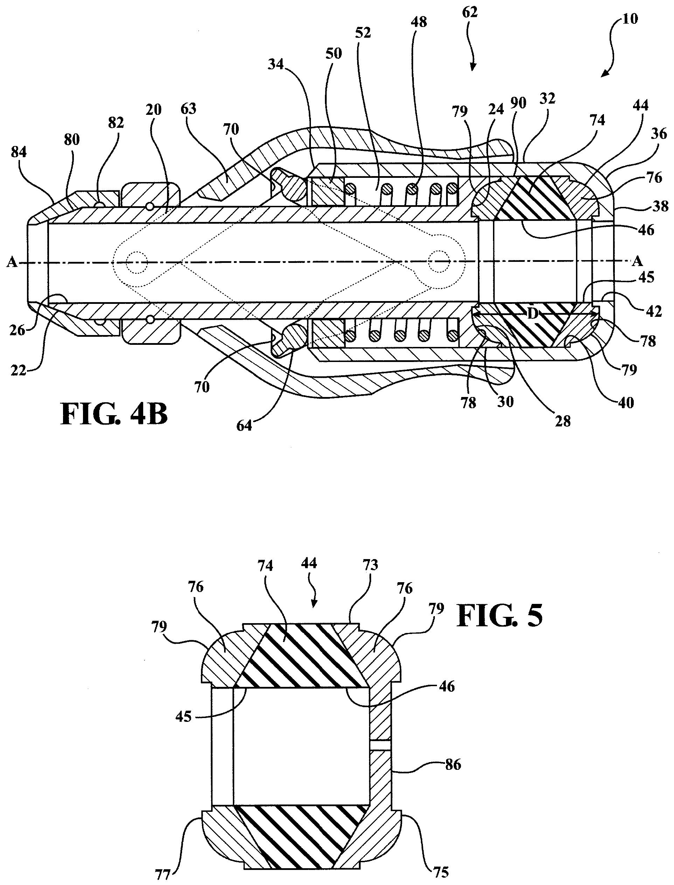

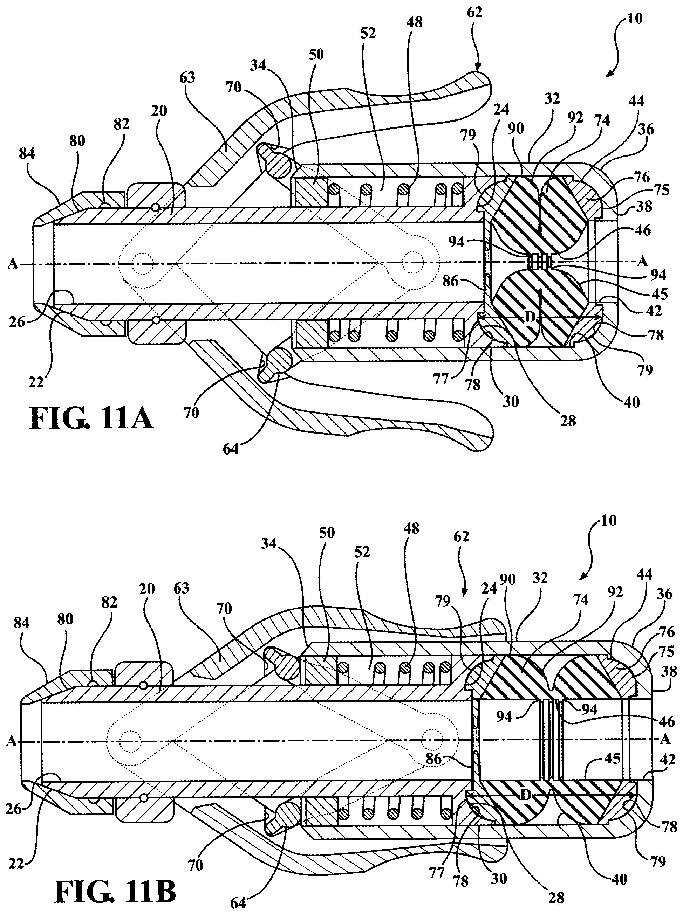

As best shown in Figures 4A, 4B, 11A and 11B, in the first embodiment of medical valve assembly **10,** the compression member **48, 54** comprises a coil spring **48** radially disposed between the valve housing **32** and the tube **20** and compressed between the first valve housing end **34** and the plunger plate **28.** However, any other suitable compression member could be utilized without departing from the scope of the subject disclosure. In a preferred embodiment, a disk **50** is slidably disposed around the tube **20** and interconnected to the first valve housing end **34** to establish a shoulder **52** extending radially inward from the valve housing **32** and which is disposed in engagement with the coil spring **48.** The coil spring **48** acts to bias the valve housing **32** towards the first tube end **22** for compressing the elastomeric seal **44** between the flange **38** and the plunger plate **28** and normally position the elastomeric seal **44** in its closed position. The valve housing **32** is then axially movable from the closed position and relative to the tube **20** to increase the distance **D** between the flange **38** and the plunger plate **28.** The increased distance **D** allows the elastomeric seal **44** to expand in an increased area of the cavity **40** disposed between the flange **38** and the plunger plate **28,** and as a result, the inner diameter **46** of the elastomeric seal **44** is expanded or increased, thereby opening the elastomeric seal **44.** The result is the establishment of the open condition of the medical valve assembly **10.**

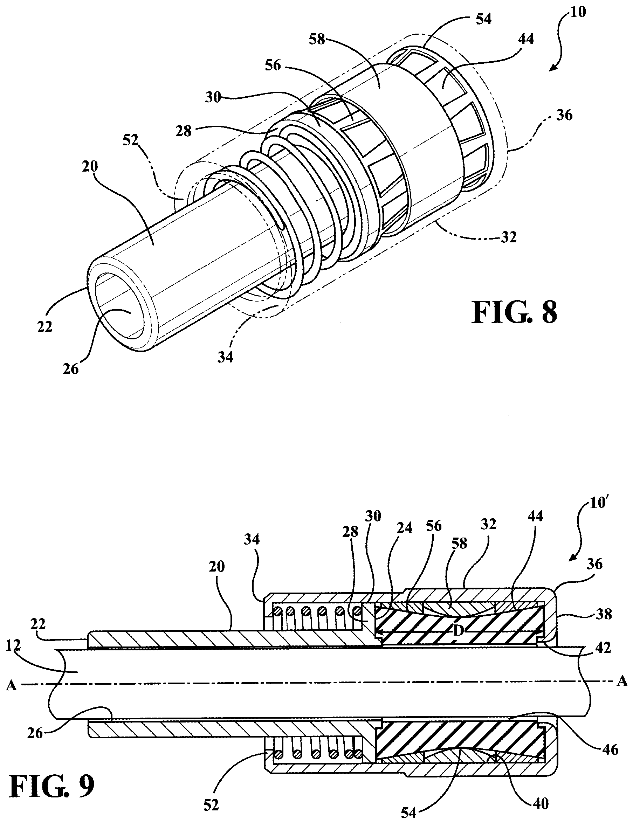

As best shown in Figures 8 and 9, in the second embodiment of the medical valve assembly **10',** the compression member **48, 54** also comprises a coil spring **48** radially disposed between the valve housing **32** and the tube **20** and compressed between the first valve housing end **34** and the plunger plate **28.** However, any other suitable compression member could be utilized without departing from the scope of the subject disclosure. The valve housing **32** defines a shoulder **52** extending radially inward from the first valve housing end **34** and slidably disposed around the tube **20**. The shoulder **52** is disposed in engagement with the coil spring **48,** and the coil spring **48** acts to bias the valve housing **36** towards the first tube end **22.** In a preferred embodiment, the compression member **48, 54** additionally includes a leaf spring cage **54** disposed in surrounding relationship with the elastomeric seal **44.** However, any other suitable compression member could be utilized without departing from the scope of the subject disclosure. The leaf spring cage **54** extends between the plunger plate **28** and the flange **38** and is compressed therebetween by way of the compression spring **48.** The leaf spring cage **54** includes a plurality of struts **56** each extending axially along the leaf spring cage **54** and configured to fold radially inward towards the elastomeric seal **44** when the valve housing **36** is axially biased towards the first tube end **22** by the compression spring **48.** As a result, the distance **D** between the plunger plate **28** and the flange **38** is decreased, thus causing the elastomeric seal **44** to compress and reduce the inner diameter **46.** Put another way, the coil spring **48** and the leaf spring cage **54** interact to compress the elastomeric seal **44** between the flange **38** and the plunger plate **28** and normally position the elastomeric seal **44** in its closed position. As a medical device **12** is inserted through the passageway **26,** the medical device **12** engages the elastomeric seal **44** with an insertion force that is transferred or exerted radially outward on the struts **56** of the leaf spring cage **54,** causing the leaf spring cage **54** to expand and counteract the biasing force of the coil spring **48.** As a result, the distance between the plunger plate **28** and the flange **38** is increased, allowing the inner diameter **46** of the elastomeric seal **44** to expand or increase and establish the open condition of the medical valve assembly **10'.** A constrictor band **58** extends around the leaf spring cage **54** to prevent the plurality of struts **56** from engaging the valve housing **32** when the leaf spring cage **54** is expanded by the insertion force of the medical device **12.**

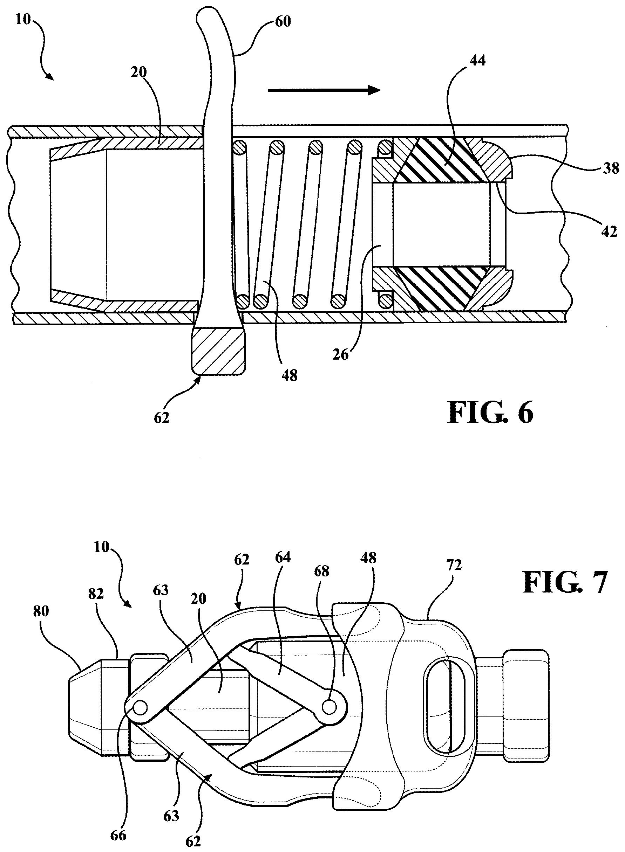

As best shown in Figures 3, 4A, 4B, 11A and 11B, the first embodiment of the medical valve assembly **10** includes a manual actuator **62** which can be connected to the valve housing **32** for allowing a user to interact with the medical valve assembly **10** and vary a size of the inner diameter **46** of the elastomeric seal **44.** Put another way, the user can interact with the manual actuator **62** to overcome the bias of the compression member **48** and move the valve housing **32** relative to the tube **20** along the axis A towards the second tube end **24.** As a result, the manual actuator **62** allows the user to manually establish the open condition of the medical valve assembly **10.** As best shown in Figure 6, in the alternative embodiment, the manual actuator **62** can include a trigger arm **60** extending radially from the valve housing **32.** In this situation, the user can pull back on the trigger arm **60** to establish the open condition of the medical valve assembly **10.** In other words, a user can pull back the trigger arm **60** to vary the bias on the plunger plate **28.**

As best shown in Figures 3, 4, 11A and 11B, the manual actuator **62** can include a pair of lever arms **63** interconnected between the tube **20** and the valve housing **32** by way of a pair of lever linkages **64.** As best shown in Figure 3, each lever arm **63** includes a first pivot **66** extending radially from the tube **20** and each lever linkage **64** includes a second pivot **68** extending radially from the valve housing **32.** The pair of lever arms **63** are pivotably connected to the tube **20** by the first pivots **66** and the pair of lever linkages **64** are pivotably connected to the valve housing by the second pivots **68** with each of the lever linkages **64** extending from the respective second pivot **68** to engage one of the respective lever arms **63.** As best shown in Figure 7, in a preferred embodiment, each of the lever arms **63** can also define a track **70** for receipt of the respective lever linkage **64** when the lever linkages **64** are disposed in abutting relationship with the lever arms **63.** This arrangement of the lever arms **63** and the lever linkages **64** allows the user to squeeze or compress the pair of lever arms **63** with a specific force to axially advance the valve housing **32** by way of the lever linkages **64.** As a result, the transferred force effectuates the increase in the distance **D** between the plunger plate **28** and the flange **38,** and thus the increase in the inner diameter **46** of the elastomeric seal **44.** Put another way, a user can radially squeeze or compress the lever arms **63** to release compression on the elastomeric seal **44** and increase the inner diameter **46** of the elastomeric seal **44** from the closed condition to a desired size of the inner diameter **46** based on an amount of radial squeeze.

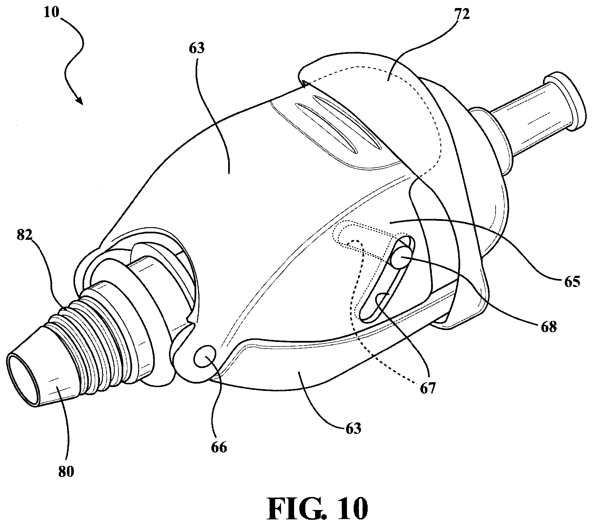

As best shown in Figure 10, in an alternative arrangement the pair of lever arms **63** can be interconnected between the tube **20** and the valve housing **32** by way of a pair of plates **65.** In a preferred embodiment, each lever arm **63** includes a plate **65** which extends radially therefrom and which defines a cam slot **67** for receiving the second pivot **68** extending radially from the valve housing **32.** This arrangement of the lever arms **63** and the plates **65** allows the user to squeeze or compress the pair of lever arms **63** with a specific force to slide the second pivot **68** along the cam slots **67** and axially advance the valve housing **32** by way of the plates **65.** As a result, the transferred force effectuates the increase in the distance **D** between the plunger plate **28** and the flange **38,** and thus the increase in the inner diameter **46** of the elastomeric seal **44.** Put another way, a user can radially squeeze or compress the lever arms **63** to release compression on the elastomeric seal **44** and increase the inner diameter **46** of the elastomeric seal **44** from the closed condition to a desired size of the inner diameter **46** based on an amount of radial squeeze.

As best shown in Figure 7, a detachable cap **72** can be snapped or disposed over the second valve housing end **36** of the valve housing **32** to hold the pair of lever arms **63** in the radially compressed position when the medical valve assembly **10** is not in use. When the detachable cap **72** is in place, it keeps the elastomeric seal **44** in the open position, and thus increases the shelf life by reducing material creep, material sticking, and/or the distorting of the elastomeric seal **44.**

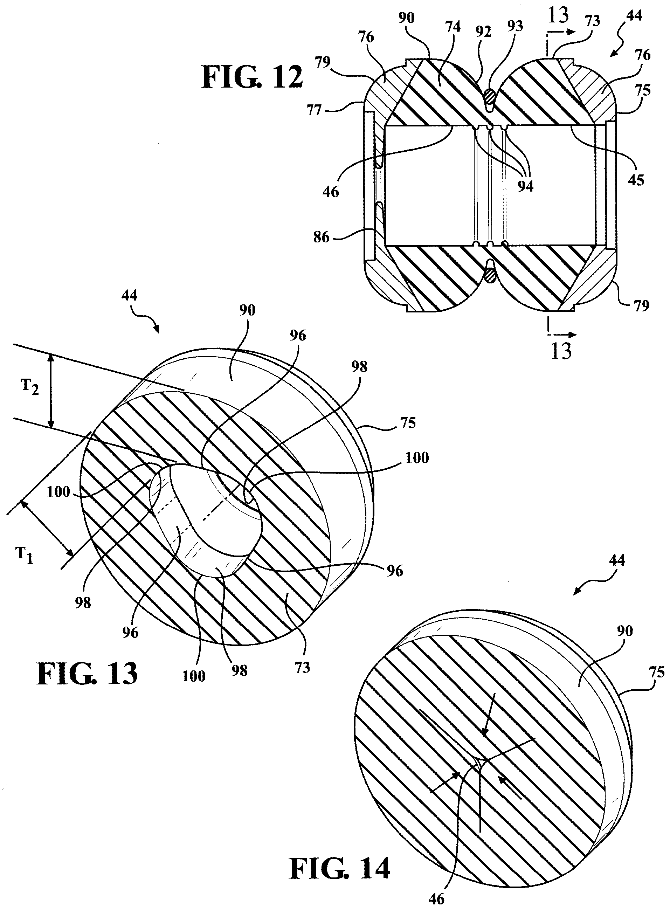

As best shown in Figures 5 and 12, the elastomeric seal **44** includes a seal body **73** which extends around the axis **A** and includes an inner portion **74** and an outer portion **76** disposed axially outwardly from the inner portion **74.** In other words, the seal body **73** extends from a proximal seal end **75** to a distal seal end **77** to define a pair of outer portions **76** disposed adjacent a respective one of the seal ends **75, 77,** each separated by the inner portion **74.** In a preferred embodiment, the inner portion **74** is made from a first material having a first durometer value and the outer portions **76** are made from a second material having a second durometer value being greater than the first durometer value. Thus, the elastomeric seal **44** preferably includes outer portions **76** that are harder than an inner portion **74.** As further shown in Figures 4A, 4B, 11A and 11B, the outer portion **76** of the elastomeric seal **44** is disposed in compressed relationship between the plunger plate **28** and the flange **38.** In a preferred embodiment, each of the pair of outer portions **76** of the elastomeric seal **44** define a curved outer surface **79** extending annularly around the axis **A** and disposed at respective proximal or distal ends **75, 77** of the elastomeric seal **44.** Correspondingly, the plunger plate **28** and the flange **38** can include curved portions **78** which are disposed in mating or engaged relationship with respective curved outer surfaces **79** of the elastomeric seal **44** to improve the retention and compression of the outer portions **76** of the elastomeric seal **44** within the medical valve assembly **10.**

As best shown in Figures 4, 11A and 11B, the tube **20** has a tapered portion **80** disposed adjacent the first tube end **22** for fitting a sheath over the tube **20**. The tube **20** includes threads **82** disposed adjacent the first tube end **22** and a nose cap **84** is threadingly secured to the first tube end **22** for establishing a compression fit of the sheath between the nose cap **84** and the tapered portion **80** of the tube **20**. Although not expressly shown, a wiper seal can be disposed within the passageway **26** between the elastomeric seal **44** and the first tube end **22** to provide a level of hemostasis around a larger device while the elastomeric seal **44** is opened for insertion of the medical device **12.** Alternatively, as best shown in Figures 5, 11A, 11B and 12, a wiper seal **86** can be incorporated into the elastomeric seal **44** and extends radially inward from one of the outer portions **76.** As best illustrated in Figure 5, in one arrangement the wiper seal **86** can extend from the outer portion **76** disposed adjacent the proximal seal end **75** of the elastomeric seal **44.** As best illustrated in Figures 11A, 11B and 12, in an alternative arrangement the wiper seal **86** extends from the outer portion **76** disposed adjacent the distal seal end **77** of the elastomeric seal **44.** As a result, when the elastomeric seal **44** is inserted in the cavity **40**, the wiper seal **86** is disposed adjacent the opening **42** of the valve housing **32.**

As best illustrated in Figures 11B and 12, the inner portion **74** of the elastomeric seal **44** includes an outer surface **90** disposed opposite the inner surface **45.** The inner portion **74** also defines an annular void **92** extending radially inwardly from the outer surface **90** and towards the inner surface **45** to remove or eliminate a portion of elastomeric material from the inner portion **74** of the elastomeric seal **44.** As such, the annular void **92** decreases the requisite compressive force to compress the elastomeric seal **44** and effectuate a closing or decreasing of the inner diameter **46,** such as illustrated in Figure 11A. This advantageously allows the inner portion **74** of the elastomeric seal **44** to be comprised of an elastomeric material having a higher or increased durometer value relative to an elastomeric seal **44** lacking the annular void **92** - which ultimately improves the durability of the elastomeric seal **44.** In a preferred arrangement, the annular void **92** has a wedge cross-sectional shape in the non-compressed condition of the elastomeric seal **44,** the cross-sectional view taken along a cross-sectional plane extending parallel to the axis **A** and between the proximal and distal seal ends **75, 77.** This "wedge" shaped annular void **92** advantageously pushes bulk material of the inner portion **74** radially inwardly towards the axis **A** as the elastomeric seal **44** is compressed to further achieve a seal or closure of the inner diameter **46** under lower compression of the elastomeric seal **44.** Put another way, the "wedge" shaped annular void **92** redirects a focal point of compression to the center of the elastomeric seal **44.** As best illustrated in Figure 12, a stability band **93** -- such as an appropriately sized o-ring -- can be disposed or seated within the annular void **92** and extends annularly around the body of the elastomeric seal **44.** The stability band **93** provides stability to the annular void **92** when medical devices are inserted or withdrawn through the elastomeric seal **44** to prevent side buckling of the elastomeric seal **44** that could result in a slight temporary leak.

As best illustrated in Figures 11A, 11B and 12, the inner portion **74** of the elastomeric seal **44** includes a plurality of ribs **94** extending radially inwardly from the inner surface **45** and encircling the axis **A** in concentric relationship with one another. During a compression of the elastomeric seal **44,** the plurality of ribs **94** disposed around the inner surface **45** will shift inwards (i.e. radially towards the axis **A)** along with the inner portion **74** and touch off sooner than the inner portion **74,** advantageously providing a seal or closure of the elastomeric seal **44** at lower compression. Furthermore, the utilization of multiple ribs **94** improves the sealing ability of the elastomeric seal **44** by providing additional sealing across each effective rib **94.** As best illustrated in Figures 11A, 11B and 12, in a preferred arrangement, the plurality of ribs **94** includes three ribs **94** extending inwardly from the inner surface **45** and radially towards the axis **A.**

As best illustrated in Figure 13, the inner surface **45** of the elastomeric seal **44** (when disposed in the non-compressed condition) includes a plurality of planar portions **96** each extending generally parallel to the axis **A** and a plurality of radiused portions **98** being convex relative to the axis **A,** with adjacent ones of the plurality of planar portions **96** being interconnected with one of the plurality of radiused portions **98.** In a preferred arrangement, the plurality of planar portions **96** includes three planar portions **96** and the plurality of radiused portions **98** includes three radiused portions **98** to define a generally triangular shape of the inner surface **45** of the elastomeric seal **44** when disposed in the non-compressed condition, the cross-sectional view taken along a plane which extends perpendicularly to the axis **A** and radially through the inner portion **74** of the elastomeric seal **44.** The inner surface **45** of the elastomeric seal **44** advantageously allows the inner surface **45** to close shut and seal under less compression (less displacement and/or force) as compared to an inner surface **45** of the elastomeric seal **44** which lacks a plurality of planar portions and a plurality of radiused portions. Furthermore, the design of the inner surface **45** with alternating planar portions **96** and radiused portions **98** improves reliability of the elastomeric seal **44** by providing a more consistent and predictable closure or sealing during an establishment of the closed condition of the medical valve assembly **10.** For example, as illustrated and highlighted in Figure 14 by the arrows, the arrangement of the inner surface **45** causes the elastomeric seal **44** to buckle or collapse radially inwardly towards the axis **A** along each of the plurality of planar portions **96.** Once the initial buckle or collapse occurs in response to a compression force exerted by the compression member **48,** the buckling or collapsing pattern is maintained throughout a compression or closure of the elastomeric seal **44,** thus providing for a predictable closure of the elastomeric seal **44.**

As further illustrated in Figure 13, the elastomeric seal **44** has a first thickness **T₁** extending between an apex **100** defined by each of the radiused portions **98** of the inner surface **45** and the outer surface **90**. The apex **100** is defined by a point along each of the radiused portions **98** which is disposed farthest radially away from the axis **A.** The elastomeric seal **44** also has a second thickness **T₂** extending between the planar portions **96** of the inner surface **45** and outer surface **90**, with the second thickness **T₂** being greater than the first thickness **T₁**. The alternating thin and thick regions of the elastomeric seal **44** as the seal body **73** extends around the axis **A** further assist in a predictable closure during compression by the compression member **48** because the thinner sections of the elastomeric seal **44** (as defined between the apex **100** of each of the radiused portions **98** and the outer surface **90**) will crease under lower compression relative to the stiffer, thick sections (as defined between the planar portions **96** and the outer surface **90**). Accordingly, the alternating thickness of the elastomeric seal **44** also improves reliability of both elastomeric seal **44** as well as the medical valve assembly **10** into which it is incorporated.

It will be appreciated by those skilled in the art that the medical valve assembly **10'** shown in Figures 8 and 9 can be equipped with the compression type manual actuator **62** shown in Figures 3, 4 and 7 or, in the alternative, the pull-type manual actuator **60** shown in Figure 6. Likewise, alternative configurations are contemplated for manual actuators that function to controllably vary the relative axial position between two components for proportionately controlling the compression load applied to an elastomeric seal to regulate an internal opening dimension defined thereby.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

A medical valve assembly as disclosed herein may be provided with an elastomeric seal that includes a plurality of ribs extending radially inwardly from said inner surface and encircling said axis A in concentric relationship with one another, wherein said plurality of ribs includes three ribs.

One of said valve housing and said tube of a medical valve assembly as described herein may be axially movable relative to the other via a manual actuator connected to said valve housing to vary the distance between said plunger plate and said flange and adjust said inner diameter of said elastomeric seal for variably sealing the medical valve assembly to a variety of differently sized medical devices.

An elastomeric seal as disclosed herein may further comprise a plurality of ribs extending radially inwardly from said inner surface and encircling said axis A in concentric relationship with one another, wherein said plurality of ribs includes three ribs.

In an elastomeric seal as disclosed herein each of said proximal and distal seal may ends of said elastomeric seal may define a curved outer surface extending annularly around said axis A.

In a medical valve as disclosed herein said elastomeric seal may include an outer portion disposed adjacent each of said proximal seal ends and an inner portion disposed between said outer portions, and said inner portion having a first durometer value and said outer portion having a second durometer value being greater than said first durometer value.

## Claims

1. A medical valve assembly (10) for use in inserting a medical device (12) into a body vessel (14) of a patient (16), comprising:
a tube (20) extending between a first tube end (22) and a second tube end (22) along an axis (A);
a plunger plate (28) extending radially from said second tube end (22) of said tube (20);
a valve housing (32) surrounding said tube (20) about said second tube end (22) and extending from a first valve housing end (34) to a second valve housing end (36);
said valve housing (32) including a flange (38) extending radially inwards from said second valve housing end (36) and disposed in spaced relationship with said plunger plate (28) to define a distance (D) extending therebetween;
an elastomeric seal (44) extending between said plunger plate (28) and said flange (38) and having an inner surface (45) defining an inner diameter (46) for use in establishing a variable seal of the medical valve assembly (10);
a coil spring (48) disposed within said valve housing (32) and compressed between said first valve housing end (34) and said plunger plate (28) for applying a compression force to said plunger plate (28) for compressing said elastomeric seal (44) from a non-compressed condition to a compressed condition to decrease said inner diameter (46) and establish a closed condition of the medical valve assembly (10);
**characterized in that**
said inner surface (45) of said elastomeric seal (44) in said non-compressed condition having a plurality of planar portions (96) and a plurality of radiused portions (98) as viewed in cross-section taken along a plane which extends perpendicularly to said axis (A) and through said elastomeric seal (44) with each of the plurality of radiused portions (98) being convex relative to said axis (A) and adjacent ones of said plurality of planar portions (96) interconnected with one of said plurality of radiused portions (98).

2. A medical valve assembly (10) as set forth in Claim 1, wherein said plurality of planar portions (96) includes three planar portions (96) and said plurality of radiused portions (100) includes three radiused portions (100) to define a generally triangular shaped inner surface as viewed in the cross-section.

3. A medical valve assembly (10) as set forth in Claim 1, further comprising:
said elastomeric seal (44) extending between a proximal seal end (75) disposed in abutting relationship with said flange (38) and a distal seal end (77) disposed in abutting relationship with said plunger plate (28);
said elastomeric seal (44) having an outer surface (90) disposed radially outwardly from said inner surface (45); and
said elastomeric seal (44) in said non-compressed condition having a first thickness (T₁) extending between an apex (100) defined by each of said plurality of radiused portions (98) and said outer surface (90) and a second thickness (T₂) extending between each of said plurality of planar portions (96) and said outer surface (90), with said second thickness (T₂) being greater than said first thickness (T₁).

4. A medical valve assembly (10) as set forth in Claim 3, wherein said elastomeric seal (44) includes a plurality of ribs (94) extending radially inwardly from said inner surface (45) and encircling said axis (A) in concentric relationship with one another.

5. A medical valve assembly (10) as set forth in Claim 4, wherein said elastomeric seal (44) defining annular void (92) extending radially inwardly from said outer surface (90) towards said inner surface (45) to decrease the requisite compression force for effectuating said decreased inner diameter (46) of said elastomeric seal (44).

6. A medical valve assembly (10) as set forth in Claim 5, wherein said annular void (92) in said non-compressed condition of said elastomeric seal (44) being wedge-shaped as viewed in a cross-section taken along a plane extending parallel to said axis (A) and between said proximal and distal seal ends (75, 77).

7. A medical valve assembly (10) as set forth in Claim 6, further comprising a stability band (93) seated within said wedge-shaped annular void (92) and extending annularly around said elastomeric seal (44).

8. A medical valve assembly (10) as set forth in Claim 3, wherein each of said proximal and distal seal ends (75, 77) of said elastomeric seal (44) define a curved outer surface (79) extending annularly around said axis (A), and wherein said plunger plate (28) and said flange (38) each define respective curved portions (78) disposed in mating or engaged relationship with a respective one of said curved outer surfaces (79) of said elastomeric seal (44) to improve a retention and compression of said elastomeric seal (44) within the medical valve assembly (10).

9. An elastomeric seal (44) for use with a medical valve assembly (10) according to claim 1, comprising:
a seal body (73) extending along an axis (A) between a proximal seal end (75) and a distal seal end (77);
said seal body (73) having an inner surface (45) extending around said axis (A) and an outer surface (90) disposed radially outwardly from said inner surface (45);
**characterized in that**
said inner surface (45) having a plurality of planar portions (96) and a plurality of radiused portions (98) as viewed in a cross-section taken along a plane which extends perpendicularly to the axis (A) and through said seal body (73) with each of the plurality of planar portions (96) extending generally parallel to said axis (A) and each of the plurality of radiused portions (98) being convex relative to said axis (A), with adjacent ones of said plurality of planar portions (96) interconnected with one of said plurality of radiused portions (98).

10. An elastomeric seal (44) as set forth in Claim 9, wherein said plurality of planar portions (96) includes three planar portions (96) and said plurality of radiused portions (98) includes three radiused portions (98) to define a generally triangular shaped inner surface of the elastomeric seal (44) as viewed in the cross-section.

11. An elastomeric seal (44) as set forth in Claim 9, wherein said seal body (73) having a first thickness (T₁) extending between an apex (100) of said plurality of radiused portions (98) and said outer surface (90) and a second thickness (T₂) extending between each of said plurality of planar portions (96) and said outer surface (90), with said second thickness (T₂) being greater than said first thickness (T₁).

12. An elastomeric seal (44) as set forth in Claim 9, further comprising a plurality of ribs (94) extending radially inwardly from said inner surface (45) and encircling said axis (A) in concentric relationship with one another.

13. An elastomeric seal (44) as set forth in Claim 12, wherein said seal body (73) defining an annular void (92) extending radially inwardly from said outer surface (90) towards said inner surface (45) to decrease a requisite compression force for compressing the elastomeric seal (4$) to effectuate a closure of the elastomeric seal (44).

14. An elastomeric seal (44) as set forth in Claim 13, wherein said annular void (92) being wedge-shaped as viewed in a cross-section taken along a plane extending parallel to said axis (A) and between said proximal and distal ends (75, 77).

15. An elastomeric seal (44) as set forth in Claim 14, further comprising a stability band (93) seated within said wedge-shaped annular void (92) and extending annularly around said seal body (73).

## Patentansprüche

1. Medizinische Ventilanordnung (10) zur Verwendung beim Einführen einer medizinischen Vorrichtung (12) in ein Körpergefäß (14) eines Patienten (16), umfassend:
ein Rohr (20), das sich zwischen einem ersten Rohrende (22) und einem zweiten Rohrende (22) entlang einer Achse (A) erstreckt;
eine Kolbenplatte (28), die sich radial von dem zweiten Rohrende (22) des Rohrs (20) erstreckt;
ein Ventilgehäuse (32), das das Rohr (20) um das zweite Rohrende (22) umgibt und sich von einem ersten Ventilgehäuseende (34) zu einem zweiten Ventilgehäuseende (36) erstreckt;
wobei das Ventilgehäuse (32) einen Flansch (38) umfasst, der sich radial nach innen von dem zweiten Ventilgehäuseende (36) erstreckt und in beabstandeter Beziehung zu der Kolbenplatte (28) angeordnet ist, um eine Distanz (D) zu definieren, der sich dazwischen erstreckt;
eine Elastomerdichtung (44), die sich zwischen der Kolbenplatte (28) und dem Flansch (38) erstreckt und eine Innenfläche (45) aufweist, die einen Innendurchmesser (46) definiert, zur Verwendung beim Herstellen einer variablen Dichtung der medizinischen Ventilanordnung (10);
eine Schraubenfeder (48), die innerhalb des Ventilgehäuses (32) angeordnet und zwischen dem ersten Ventilgehäuseende (34) und der Kolbenplatte (28) komprimiert ist, um eine Kompressionskraft auf die Kolbenplatte (28) auszuüben, um die Elastomerdichtung (44) von einem nicht komprimierten Zustand in einen komprimierten Zustand zu komprimieren, um den Innendurchmesser (46) zu verringern und einen geschlossenen Zustand der medizinischen Ventilanordnung (10) herzustellen;
**dadurch gekennzeichnet, dass**
die Innenfläche (45) der Elastomerdichtung (44) in dem nicht komprimierten Zustand eine Vielzahl von ebenen Abschnitten (96) und eine Vielzahl von abgerundeten Abschnitten (98) bei Betrachtung im Querschnitt entlang einer Ebene aufweist, die sich senkrecht zu der Achse (A) und durch die Elastomerdichtung (44) erstreckt, wobei jeder der Vielzahl von abgerundeten Abschnitten (98) relativ zu der Achse (A) konvex ist und benachbarte der Vielzahl von ebenen Abschnitten (96) mit einem der Vielzahl von abgerundeten Abschnitten (98) verbunden sind.

2. Medizinische Ventilanordnung (10) nach Anspruch 1, wobei die Vielzahl von ebenen Abschnitten (96) drei ebene Abschnitte (96) umfasst und die Vielzahl von abgerundeten Abschnitten (100) drei abgerundete Abschnitte (100) umfasst, um bei Betrachtung im Querschnitt eine im Allgemeinen dreieckig geformte Innenfläche zu definieren.

3. Medizinische Ventilanordnung (10) nach Anspruch 1, ferner umfassend, dass:
sich die Elastomerdichtung (44) sich zwischen einem proximalen Dichtungsende (75), das in angrenzender Beziehung zu dem Flansch (38) angeordnet ist, und einem distalen Dichtungsende (77) erstreckt, das in angrenzender Beziehung zu der Kolbenplatte (28) angeordnet ist;
die Elastomerdichtung (44) eine Außenfläche (90) aufweist, die radial nach außen von der Innenfläche (45) angeordnet ist; und
die Elastomerdichtung (44) in dem nicht komprimierten Zustand eine erste Dicke (T₁), die sich zwischen einem Scheitelpunkt (100) erstreckt, der durch jeden der Vielzahl von abgerundeten Abschnitten (98) und der Außenfläche (90) definiert ist, und eine zweite Dicke (T₂) aufweist, die sich zwischen jedem der Vielzahl von ebenen Abschnitten (96) und der Außenfläche (90) erstreckt, wobei die zweite Dicke (T₂) größer als die erste Dicke (T₁) ist.

4. Medizinische Ventilanordnung (10) nach Anspruch 3, wobei die Elastomerdichtung (44) eine Vielzahl von Rippen (94) umfasst, die sich radial nach innen von der Innenfläche (45) erstrecken und die Achse (A) in konzentrischer Beziehung zueinander umgeben.

5. Medizinische Ventilanordnung (10) nach Anspruch 4, wobei die Elastomerdichtung (44) einen ringförmigen Hohlraum (92) definiert, der sich radial nach innen von der Außenfläche (90) zu der Innenfläche (45) erstreckt, um die erforderliche Kompressionskraft zum Bewirken des verringerten Innendurchmessers (46) der Elastomerdichtung (44) zu verringern.

6. Medizinische Ventilanordnung (10) nach Anspruch 5, wobei der ringförmige Hohlraum (92) in dem nicht komprimierten Zustand der Elastomerdichtung (44) bei Betrachtung in einem Querschnitt entlang einer Ebene keilförmig ist, die sich parallel zu der Achse (A) und zwischen dem proximalen und distalen Dichtungsende (75, 77) erstreckt.

7. Medizinische Ventilanordnung (10) nach Anspruch 6, ferner umfassend ein Stabilitätsband (93), das in dem keilförmigen ringförmigen Hohlraum (92) sitzt und sich ringförmig um die Elastomerdichtung (44) erstreckt.

8. Medizinische Ventilanordnung (10) nach Anspruch 3, wobei jedes von dem proximalen und distalen Dichtungsende (75, 77) der Elastomerdichtung (44) eine gekrümmte Außenfläche (79) definiert, die sich ringförmig um die Achse (A) erstreckt, und wobei die Kolbenplatte (28) und der Flansch (38) jeweils jeweilige gekrümmte Abschnitte (78) definieren, die in passender oder in Eingriff stehender Beziehung zu einer jeweiligen der gekrümmten Außenflächen (79) der Elastomerdichtung (44) angeordnet sind, um ein Halten und Komprimieren der Elastomerdichtung (44) innerhalb der medizinischen Ventilanordnung (10) zu verbessern.

9. Elastomerdichtung (44) zur Verwendung mit einer medizinischen Ventilanordnung (10) nach Anspruch 1, umfassend:
einen Dichtungskörper (73), der sich entlang einer Achse (A) zwischen einem proximalen Dichtungsende (75) und einem distalen Dichtungsende (77) erstreckt;
wobei der Dichtungskörper (73) eine Innenfläche (45), die sich um die Achse (A) erstreckt, und eine Außenfläche (90) aufweist, die radial nach außen von der Innenfläche (45) angeordnet ist;
**dadurch gekennzeichnet, dass**
die Innenfläche (45) eine Vielzahl von ebenen Abschnitten (96) und eine Vielzahl von abgerundeten Abschnitten (98) aufweist, wie in einem Querschnitt entlang einer Ebene betrachtet, die sich senkrecht zu der Achse (A) und durch den Dichtungskörper (73) erstreckt, wobei jeder der Vielzahl von ebenen Abschnitten (96) sich im Allgemeinen parallel zu der Achse (A) erstreckt und jeder der Vielzahl von abgerundeten Abschnitten (98) relativ zu der Achse (A) konvex ist, wobei benachbarte der Vielzahl von ebenen Abschnitten (96) mit einem der Vielzahl von abgerundeten Abschnitten (98) verbunden sind.

10. Elastomerdichtung (44) nach Anspruch 9, wobei die Vielzahl von ebenen Abschnitten (96) drei ebene Abschnitte (96) umfasst und die Vielzahl von abgerundeten Abschnitten (98) drei abgerundete Abschnitte (98) umfasst, um eine bei Betrachtung im Querschnitt im Allgemeinen dreieckig geformte Innenfläche der Elastomerdichtung (44) zu definieren.

11. Elastomerdichtung (44) nach Anspruch 9, wobei der Dichtungskörper (73) eine erste Dicke (T₁), die sich zwischen einem Scheitelpunkt (100) der Vielzahl von abgerundeten Abschnitten (98) und der Außenfläche (90) erstreckt, und eine zweite Dicke (T₂) aufweist, die sich zwischen jedem der Vielzahl von ebenen Abschnitten (96) und der Außenfläche (90) erstreckt, wobei die zweite Dicke (T₂) größer als die erste Dicke (T₁) ist.

12. Elastomerdichtung (44) nach Anspruch 9, ferner umfassend eine Vielzahl von Rippen (94), die sich radial nach innen von der Innenfläche (45) erstrecken und die Achse (A) in konzentrischer Beziehung zueinander umgeben.

13. Elastomerdichtung (44) nach Anspruch 12, wobei der Dichtungskörper (73) einen ringförmigen Hohlraum (92) definiert, der sich radial nach innen von der Außenfläche (90) zu der Innenfläche (45) erstreckt, um eine erforderliche Kompressionskraft zum Komprimieren der Elastomerdichtung (44) zu verringern, um ein Schließen der Elastomerdichtung (44) zu bewirken.

14. Elastomerdichtung (44) nach Anspruch 13, wobei der ringförmige Hohlraum (92) bei Betrachtung in einem Querschnitt entlang einer Ebene keilförmig ist, die sich parallel zu der Achse (A) und zwischen dem proximalen und distalen Ende (75, 77) erstreckt.

15. Elastomerdichtung (44) nach Anspruch 14, ferner umfassend ein Stabilitätsband (93), das in dem keilförmigen ringförmigen Hohlraum (92) sitzt und sich ringförmig um den Dichtungskörper (73) erstreckt.

## Revendications

1. Assemblage de valvule médicale (10) utilisé pour insérer un dispositif médical (12) jusque dans un vaisseau corporel (14) d'un patient (16), comprenant :
un tube (20) s'étendant entre une première extrémité de tube (22) et une seconde extrémité de tube (22) le long d'un axe (A) ;
une plaque de piston (28) s'étendant radialement depuis ladite seconde extrémité de tube (22) dudit tube (20) ;
un boîtier de valvule (32) entourant ledit tube (20) autour de ladite seconde extrémité de tube (22) et s'étendant depuis une première extrémité de boîtier de valvule (34) jusqu'à une seconde extrémité de boîtier de valvule (36) ;
ledit boîtier de valvule (32) incluant une bride (38) s'étendant radialement vers l'intérieur depuis ladite seconde extrémité de boîtier de valvule (36) et disposée dans une relation d'espacement avec ladite plaque de piston (28) pour définir une distance (D) s'étendant entre les deux ;
un joint d'étanchéité élastomère (44) s'étendant entre ladite plaque de piston (28) et ladite bride (38) et ayant une surface intérieure (45) définissant un diamètre intérieur (46) utilisé pour établir une étanchéité variable de l'assemblage de valvule médicale (10) ;
un ressort à boudin (48) disposé à l'intérieur dudit boîtier de valvule (32) et comprimé entre ladite première extrémité de boîtier de valvule (34) et ladite plaque de piston (28) pour appliquer une force de compression à ladite plaque de piston (28) afin de comprimer ledit joint d'étanchéité élastomère (44) depuis un état non comprimé jusque dans un état comprimé pour diminuer ledit diamètre intérieur (46) et réaliser un état fermé de l'assemblage de valvule médicale (10) ;
**caractérisé en ce que**
ladite surface intérieure (45) dudit joint d'étanchéité élastomère (44) dans ledit état non comprimé a une pluralité de portions planaires (96) et une pluralité de portions arrondies (98) dans une vue en section transversale prise le long d'un plan qui s'étend perpendiculairement audit axe (A) et à travers ledit joint d'étanchéité élastomère (44), chacune de la pluralité de portions arrondies (98) étant convexe relativement à l'axe (A) et des portions adjacentes de ladite pluralité de portions planaires (96) étant interconnectées avec l'une de ladite pluralité de portions arrondies (98).

2. Assemblage de valvule médicale (10) selon la revendication 1, dans lequel ladite pluralité de portions planaires (96) incluent trois portions planaires (96) et ladite pluralité de portions arrondies (98) incluent trois portions arrondies (98) pour définir une surface intérieure de forme généralement triangulaire dans une vue en section transversale.

3. Assemblage de valvule médicale (10) selon la revendication 1, comprenant en outre :
ledit joint d'étanchéité élastomère (44) s'étendant entre une extrémité de joint d'étanchéité proximale (75) disposée dans une relation de butée avec ladite bride (38) et une extrémité de joint d'étanchéité distale (77) disposée dans une relation de butée avec ladite plaque de piston (28) ;
ledit joint d'étanchéité élastomère (44) ayant une surface extérieure (90) disposée radialement à l'extérieur de ladite surface intérieure (45) ; et
ledit joint d'étanchéité élastomère (44) dans ledit état non comprimé ayant une première épaisseur (T₁) s'étendant entre un sommet (100) défini par chacune de ladite pluralité de portions arrondies (98) et ladite surface extérieure (90), et une seconde épaisseur (T₂) s'étendant entre chacune de ladite pluralité de portions planaires (96) et ladite surface extérieure (90), ladite seconde épaisseur (T₂) étant supérieure à ladite première épaisseur (T₁).

4. Assemblage de valvule médicale (10) selon la revendication 3, dans lequel ledit joint d'étanchéité élastomère (44) inclut une pluralité de nervures (94) s'étendant radialement vers l'intérieur depuis ladite surface intérieure (45) et encerclant ledit axe (A) dans une relation de concentricité les unes par rapport aux autres.

5. Assemblage de valvule médicale (10) selon la revendication 4, dans lequel ledit joint d'étanchéité élastomère (44) définit un vide annulaire (92) s'étendant radialement vers l'extérieur depuis ladite surface extérieure (90) vers ladite surface intérieure (45) pour diminuer la force de compression requise afin de réaliser ladite diminution du diamètre intérieur (46) dudit joint d'étanchéité élastomère (44).

6. Assemblage de valvule médicale (10) selon la revendication 5, dans lequel ledit vide annulaire (92) dans ledit état non comprimé dudit joint d'étanchéité élastomère (44) est en forme de coin dans une vue en section transversale prise le long d'un plan s'étendant parallèlement audit axe (A) et entre lesdites extrémités de joint d'étanchéité proximale et distale (75, 77).

7. Assemblage de valvule médicale (10) selon la revendication 6, comprenant en outre une bande de stabilité (93) en assise à l'intérieur du vide annulaire en forme de coin (92) et s'étendant de manière annulaire autour dudit joint d'étanchéité élastomère (44).

8. Assemblage de valvule médicale (10) selon la revendication 3, dans lequel chacune desdites extrémités de joint d'étanchéité proximale et distale (75, 77) dudit joint d'étanchéité élastomère (44) définit une surface extérieure incurvée (79) s'étendant de manière annulaire autour dudit axe (A), et dans lequel ladite plaque de piston (28) et ladite bride (38) définissent chacune des portions incurvées respectives (78) disposées dans une relation d'appariement ou d'engagement avec une surface respective desdites surfaces extérieures incurvées (79) dudit joint d'étanchéité élastomère (44) pour améliorer une rétention et une compression dudit joint d'étanchéité élastomère (44) avec l'assemblage de valvule médicale (10).

9. Joint d'étanchéité élastomère (44) destiné à être utilisé avec un assemblage de valvule médicale (10) selon la revendication 1, comprenant :
un corps de joint d'étanchéité (73) s'étendant le long d'un axe (A) entre une extrémité de joint d'étanchéité proximale (75) et une extrémité de joint d'étanchéité distale (77) ;
ledit corps de joint d'étanchéité (73) ayant une surface intérieure (45) s'étendant autour dudit axe (A) et une surface extérieure (90) disposée radialement à l'extérieur de ladite surface intérieure (45) ;
**caractérisé en ce que**
ladite surface intérieure (45) a une pluralité de portions planaires (96) et une pluralité de portions arrondies (98) dans une vue en section transversale prise le long d'un plan qui s'étend perpendiculairement à l'axe (A) et à travers ledit corps de joint d'étanchéité (73), chaque portion de la pluralité de portions planaires (96) s'étendant généralement parallèlement audit axe (A) et chacune de la pluralité de portions arrondies (98) étant convexe relativement audit axe (A), des portions adjacentes de ladite pluralité de portions planaires (96) étant interconnectées avec l'une de ladite pluralité de portions arrondies (98).

10. Joint d'étanchéité élastomère (44) selon la revendication 9, dans lequel ladite pluralité de portions planaires (96) incluent trois portions planaires (96) et ladite pluralité de portions arrondies (98) incluent trois portions arrondies (98) pour définir une surface intérieure de forme généralement triangulaire du joint d'étanchéité élastomère (44) dans une vue en section transversale.

11. Joint d'étanchéité élastomère (44) selon la revendication 9, dans lequel ledit corps de joint d'étanchéité a une première épaisseur (T₁) s'étendant entre un sommet (100) de ladite pluralité de portions arrondies (98) et ladite surface extérieure (90), et une seconde épaisseur (T₂) s'étendant entre chacune de ladite pluralité de portions planaires (96) et ladite surface extérieure (90), ladite seconde épaisseur (T₂) étant supérieure à ladite première épaisseur (T₁).

12. Joint d'étanchéité élastomère (44) selon la revendication 9, comprenant en outre une pluralité de nervures s'étendant radialement vers l'intérieur depuis ladite surface intérieure (45) et encerclant ledit axe (A) dans une relation de concentricité les unes par rapport aux autres.

13. Joint d'étanchéité élastomère (44) selon la revendication 12, dans lequel ledit corps de joint d'étanchéité (73) définit un vide annulaire (92) s'étendant radialement vers l'intérieur depuis ladite surface extérieure (90) vers ladite surface intérieure (45) pour diminuer une force de compression requise afin de comprimer le joint d'étanchéité élastomère (44) pour réaliser une fermeture du joint d'étanchéité élastomère (44).

14. Joint d'étanchéité élastomère (44) selon la revendication 13, dans lequel ledit vide annulaire (92) est en forme de coin dans une vue en section transversale prise le long d'un plan s'étendant parallèlement audit axe (A) et entre lesdites extrémités proximale et distale (75, 77).

15. Joint d'étanchéité élastomère (44) selon la revendication 14, comprenant en outre une bande de stabilité (93) en assise à l'intérieur dudit vide annulaire en forme de coin (92) et s'étendant de manière annulaire autour dudit corps de joint d'étanchéité (73).
